# EUROPÄISCHE PATENTSCHRIFT

(11) **EP 2 563 285 B1**
(45) Veröffentlichungstag und Bekanntmachung des Hinweises auf die Patenterteilung: **17.12.2014**
(21) Anmeldenummer: 11717959.8
(22) Anmeldetag: 28.04.2011
(51) Int. Cl.: A61F 2/82, A61L 31/02

(54) **VERFAHREN ZUR HERSTELLUNG EINER MEDIZINISCHEN VORRICHTUNG**
METHOD FOR PRODUCING A MEDICAL DEVICE
PROCÉDÉ DE FABRICATION D'UN DISPOSITIF MÉDICAL

(30) Priorität: 28.04.2010 DE 102010018541
(43) Veröffentlichungstag der Anmeldung: 06.03.2013
(73) Patentinhaber: Acandis GmbH & Co. KG, 76327 Pfinztal (DE)
(72) Erfinder: ZAMPONI, Christiane, 24114 Kiel (DE); LIMA DE MIRANDA, Rodrigo, 24103 Kiel (DE)
(74) Vertreter: Kilchert, Jochen
(86) Internationale Anmeldenummer: PCT/EP2011/002137
(87) Internationale Veröffentlichungsnummer: WO 2011/134662

(56) Entgegenhaltungen:
- WO-A2-2007/093423
- WO-A2-2008/022799
- DE-A1-102006 029 831

## Beschreibung

Die Erfindung betrifft ein Verfahren zur Herstellung einer medizinischen Vorrichtung.

Für die Behandlung von Gefäßläsionen, insbesondere in zerebralen Gefäßen, mit Stents bzw. allgemein mit rohrförmigen, d.h. dreidimensionalen Strukturen, ist es zweckmäßig, wenn diese eine relativ hohe Flexibilität aufweisen, um diese Strukturen in stark gekrümmten Gefäßen bewegen bzw. platzieren zu können. Bei gitterförmigen Strukturen werden diese durch Krümmung in axiale Richtung verlängert, wobei sich die Bereiche der Strukturen, die sich auf der Außenseite der Krümmung befinden, weiter dehnen bzw. strecken, als diejenigen Bereiche, die näher am Krümmungsmittelpunkt angeordnet sind. Es besteht also ein Zusammenhang zwischen der Flexibilität im Sinne der Krümmungs- bzw. Biegefähigkeit und der Längenänderung bzw. maximalen Verlängerung der gitterförmigen Struktur.

Aufgrund der kleinen Dimensionen der gitterförmigen Strukturen, insbesondere der Stents, die sich im unteren Mikrometerbereich befinden, ist es vorteilhaft, wenn die vom Stent bzw. von der gitterförmigen Struktur auf die Gefäßwand übertragene Radialkraft möglichst gut verteilt wird, wobei gleichzeitig die Flexibilität erhalten werden soll. Ferner ist für das Einbringen der gitterförmigen Struktur in den Körper mittels eines Katheters die Crimpbarkeit der Struktur wichtig. Gitterförmige Strukturen, die die vorstehend genannten Anforderungen an die Flexibilität, die Crimpbarkeit und die Radialkraftverteilung erfüllen, können beispielsweise dadurch hergestellt werden, dass auf die Elemente der Gitterstrukturen, insbesondere auf die Stege, eine Abdeckung aufgebracht wird, die etwas breiter als die Stegbreite ist. Im Querschnitt kann sich dabei ein T-förmiges Profil ergeben, das sich einerseits aus dem Steg und andererseits aus der senkrecht zum Steg erstreckten Abdeckung zusammensetzt.

Andere Anwendungsfälle, bei denen auf eine gitterförmige Stützstruktur eine Abdeckung, insbesondere eine strukturierte Abdeckung aufgebracht wird, sind bekannt, beispielsweise im Zusammenhang mit der Herstellung von Thrombosefiltern, die bei der Behandlung von Stenosen eingesetzt werden, um zu verhindern, dass sich thrombotisches Material von den Gefäßwänden löst und in den Blutkreislauf gelangt.

Ein derartiges Herstellungsverfahren ist beispielsweise aus WO 2008/022799 A2 bekannt. Bei dem bekannten Verfahren wird eine Gitterstruktur auf ein Substrat aufgebracht, wobei das Substrat und die Gitterstruktur durch einen Haftvermittler verbunden werden, so dass die Öffnungen der Gitterstruktur durch das Substrat einigermaßen dicht verschlossen sind. Die Öffnungen der Gitterstruktur werden über die unbedeckte Seite mit einem Opfermaterial befüllt, so dass sich nach Entfernen des Substrats auf der vorher mit dem Substrat verbundenen Seite eine relativ glatte geschlossene Oberfläche ergibt. Auf dieser wird dann die Abdeckschicht abgeschieden, die nach Entfernen des Opfermaterials strukturiert werden kann. Auf diese Weise können die gewünschten Strukturen einfach und kostengünstig hergestellt werden.

Allerdings kann es bei der Herstellung der Gitterstruktur zu Schwankungen der Wandstärke bzw. Breite der einzelnen Strukturelemente, bspw. der Stege kommen. Außerdem können die Strukturelemente unregelmäßige, insbesondere abgerundete Kanten aufweisen, die beim Elektropolieren der Strukturelemente entstehen. Diese Konturen der Gitterelemente können die Aufbringung und Anhaftung der Abdeckung an den Gitterelementen beeinträchtigen.

Der Erfindung liegt die Aufgabe zu Grunde, ein Verfahren zur Herstellung einer medizinischen Vorrichtung mit einer Gitterstruktur anzugeben, die zumindest abschnittsweise mit einer Abdeckung verbunden ist, wobei die Verbindung zwischen der Gitterstruktur und der Abdeckung verbessert wird.

Erfindungsgemäß wird diese Aufgabe durch ein Verfahren mit den Merkmalen des Anspruchs 1 gelöst.

Die Erfindung beruht auf dem Gedanken, ein Verfahren zur Herstellung einer medizinischen Vorrichtung mit einer Gitterstruktur anzugeben, die zumindest abschnittsweise mit einer Abdeckung verbunden ist, wobei das Verfahren die folgenden Schritte aufweist:
Die Gitterstruktur wird mit der Seite, die mit der Abdeckung zu verbinden ist, auf einem Substrat angeordnet. Die Gitterstruktur wird mit der auf dem Substrat angeordneten Seite in das Substrat zumindest teilweise eingedrückt. Die Gitterstruktur wird mit einer ersten Schicht aus einem Opfermaterial beschichtet, so dass zumindest ein Teil der Zwischenräume der Gitterstruktur mit der ersten Schicht bedeckt wird und die erste Schicht mit der Gitterstruktur auf der mit der Abdeckung zu verbindenden Seite eine im Wesentlichen geschlossene Fläche bildet. Das bedeutet, dass die Gitterstruktur zumindest teilweise mit dem Opfermaterial der ersten Schicht aufgefüllt wird. Das Substrat wird entfernt, so dass die Seite der Gitterstruktur zugänglich ist, die mit der Abdeckung zu verbinden ist. Eine zweite Schicht zur Bildung der Abdeckung wird auf die zuvor in das Substrat eingedrückte Seite der Gitterstruktur aufgebracht. Das Opfermaterial wird entfernt.

Die so erhältliche medizinische Vorrichtung weist eine tragende Gitterstruktur auf, die zumindest abschnittsweise mit einer Abdeckung verbunden ist, wobei die Verbindung zwischen der Gitterstruktur und der Abdeckung im Wesentlichen unabhängig von etwaigen Konturschwankungen der einzelnen Elemente der Gitterstruktur ist.

Im Unterschied zu dem Verfahren gemäß WO 2008/022799 A2, bei dem die Gitterstruktur plan auf dem Substrat aufliegt, wird bei dem erfindungsgemäßen Verfahren die Gitterstruktur nach dem Aufbringen in das Substrat aktiv eingedrückt. Damit wird erreicht, dass die Konturen der einzelnen Gitterelemente der Gitterstruktur im Substrat abgebildet werden derart, dass das Substrat einen Teil der Gitterelemente, nämlich die kritischen Bereiche, insbesondere die Kantenbereiche abdeckt. Die Gitterelemente kontaktieren das Substrat auf der mit der Abdeckung zu verbindenden Seite vollständig, so dass die Bildung von Schattenstellen vermieden wird. Die erste Schicht aus dem Opfermaterial, die die Zwischenräume bzw. Öffnungen der Gitterstruktur ausfüllt, bedeckt somit den substratfreien Teil der Gitterelemente, also die unkritischen Bereiche, die bei der Herstellung der Gitterelemente nicht oder weniger stark Konturschwankungen unterliegen. Damit wird ein möglichst vollständiger Kontakt der ersten Schicht aus dem Opfermaterial und der Gitterstruktur erreicht.

Nach dem Ablösen des Substrats stehen die zuvor vom Substrat bedeckten Bereiche der Gitterstruktur, also die kritischen Bereiche der Gitterstruktur über die erste Schicht aus dem Opfermaterial etwas hervor und zwar entsprechend der Eindrücktiefe der Gitterstruktur in das Substrat. Beim nachfolgenden Aufbringen der zweiten Schicht zur Bildung der Abdeckung kann deshalb die zweite Schicht die vorstehenden Bereiche der Gitterstruktur unabhängig von deren Kontur bzw. von etwaigen Konturschwankungen vollständig bzw. nahezu vollständig bedecken. Dadurch wird die Anhaftung der Abdeckung auf der Gitterstruktur im Sinne einer dauerhaften festen Verbindung verbessert. Die Eindrücktiefe kann mindestens in einem Bereich von 30 nm bis 200 nm eingestellt werden (Bereich für die Untergrenze), d.h. die Untergrenze kann mindestens 30 nm, insbesondere mindestens 40 nm, insbesondere mindestens 50 nm, insbesondere mindestens 75 nm, insbesondere mindestens 100 nm, insbesondere mindestens 125 nm, insbesondere mindestens 150 nm, insbesondere mindestens 175 nm, insbesondere mindestens 200 nm, insbesondere mindestens 225 nm, insbesondere mindestens 250 nm, insbesondere mindestens 275 nm, insbesondere mindestens 300 nm, insbesondere mindestens 350 nm, insbesondere mindestens 400 nm betragen. Dadurch wird eine gute Anhaftung der Abdeckung an den Gitterelementen erreicht und das Aufwachsen der zweiten Schicht, insbesondere des Kupfers unter den Gitterelementen wird vermieden. Die maximale Eindrücktiefe wird in einem Bereich von 0,4 bis 2 µm eingestellt (Bereich für die Obergrenze), d.h. höchstens 2 µm, insbesondere höchstens 1 µm, insbesondere höchstens 0,8 µm, insbesondere höchstens 0,6 µm, insbesondere höchstens 0,4 µm,. Damit wird eine übermäßige Konturierung der Abdeckung bzw. Folie und eine mögliche Ausbildung eines Kanals zwischen Abdeckung und Gitterelementen vermieden. Der Gesamtbereich beträgt ca. 30 nm bis 2 µm.

Die Eindrücktiefe bzw. die Eindringtiefe der Gitterstruktur in das Substrat kann vom Fachmann in Abhängigkeit von der Beschaffenheit der Gitterstruktur im Bereich der Kanten der Gitterelemente gewählt werden. Je tiefer die Gitterstruktur in das Substrat eingedrückt wird, umso weiter bedeckt die zweite Schicht zur Bildung der Abdeckung die seitlichen Bereiche der Gitterelemente, die im Wesentlichen senkrecht zur Außenfläche der Gitterstruktur angeordnet sind, die im Gebrauch bzw. im expandierten Zustand an der Gefäßwand anliegt und Radialkräfte überträgt.

Bei einer bevorzugten Ausführungsform des erfindungsgemäßen Verfahrens wird die Gitterstruktur derart in das Substrat eingedrückt, dass Kanten oder seitliche Abrundungen der Gitterstruktur im Substrat eingebettet sind. Dadurch wird erreicht, dass die für die Verbindung zwischen der Gitterstruktur und der Abdeckung kritischen Bereiche der Gitterstruktur nach dem Entfernen des Substrats mit der zweiten Schicht zur Bildung der Abdeckung verbunden werden können.

Das Verhältnis zwischen dem in das Substrat eingedrückten Teil der Gitterstruktur und dem außerhalb des Substrats befindlichen Teil der Gitterstruktur kann mindestens 0,0005, insbesondere mindestens 0,001, insbesondere mindestens 0,005, insbesondere mindestens 0,01, insbesondere mindestens 0,03, insbesondere mindestens 0,05, insbesondere mindestens 0,07, insbesondere mindestens 0,1, insbesondere mindestens 0,15, insbesondere mindestens 0,2 betragen. Das vorstehend genannte Verhältnis zwischen eingedrücktem und freiem Teil der Gitterstege kann bspw. auf Stege mit einer Stegdicke von 30µm bis 60µm und/oder auf die vorstehend genannten Bereich für die Ober- und Untergrenzen der Eindrücktiefe bezogen werden.

Das Eindrücken der Gitterstruktur in das Substrat kann dadurch begünstigt werden, dass das Substrat flexibel ist und/oder eine Außenschicht aus einem flexiblen Material, insbesondere eine Außenschicht aus Fotolack oder einem viskosen Polymer aufweist, die beim Eindrücken des Substrats nachgibt. Die Außenschicht aus einem Fotolack oder dem viskosen Polymer hat den Vorteil, dass die Gitterstruktur auf dem Substrat aufgrund der Klebeeigenschaften des Fotolacks fixiert wird.. Ein anderer Vorteil ist zum einem die Ätzselektivität und zum anderen die Möglichkeit, auf dieser Außenschicht eine Startschicht für den Galvanikprozess abzuscheiden.
Zum Entfernen des Substrats kann es ausreichend sein, die Außenschicht des Substrats zu entfernen, wodurch das Ablösen des Substrats erleichtert wird.

Die Beschichtung der Gitterstruktur mit der ersten Schicht aus einem Opfermaterial kann dadurch verbessert werden, dass die Gitterstruktur vor dem Beschichten mit der ersten Schicht mit einer Startschicht beschichtet wird. Vorzugsweise ist das Opfermaterial der ersten Schicht und/oder die Startschicht aus einem metallischen Material, insbesondere aus demselben metallischen Material oder aus unterschiedlichen metallischen Materialien gebildet.

Das Beschichten der Gitterstruktur mit der Startschicht kann durch ein Gasphasenabscheidungsverfahren, insbesondere ein PVD-Verfahren, insbesondere durch Sputtern und/oder das Beschichten der Gitterstruktur mit der ersten Schicht aus einem Opfermaterial durch ein galvanisches Verfahren erfolgen. Die Aufbringung der Startschicht durch ein Gasphasenabscheidungsverfahren hat den Vorteil, dass die Gitterstruktur mit einer dünnen Startschicht bedeckt werden kann. Durch das Gasphasenabscheidungsverfahren wird eine gleichmäßige Beschichtung der Gitterstruktur und des Substrats erreicht, so dass die Bildung von Hohlräumen bei der nachfolgenden Behandlung vermieden wird. Außerdem verbessert die Startschicht die nachfolgende vollständige Auffüllung der Gitterstruktur. Für die Auffüllung der Zwischenräume zwischen den einzelnen Elementen der Gitterstruktur eignet sich besonders gut ein galvanisches Verfahren, mit dem in relativ kurzer Zeit eine ausreichend große Materialmenge auf bzw. in der Gitterstruktur abgeschieden wird. Es ist auch möglich, die erste Schicht aus dem Opfermaterial vollständig durch das Gasphasenabscheidungsverfahren aufzubringen, also die Auffüllung der Zwischenräume bzw. Öffnungen der Gitterstruktur durch Sputtern zu erreichen.

Bei einer weiteren besonders bevorzugten Ausführungsform wird die zweite Schicht zur Bildung der Abdeckung durch ein Gasphasenabscheidungsverfahren, insbesondere ein PVD-Verfahren, insbesondere durch Sputtern aufgebracht. Auf diese Weise lassen sich dünne Schichten zur Bildung der Abdeckung erzeugen (Dünnschichttechnologie). Die zweite Schicht zur Bildung der Abdeckung kann nach dem Aufbringen strukturiert werden, insbesondere durch ein lithografisches Verfahren. Damit kann die Abdeckung an den jeweiligen Einsatzzweck angepasst werden. Beispielsweise können durch die Strukturierung der zweiten Schicht im Querschnitt T-förmige Profile der einzelnen Gitterelemente erzeugt werden, so dass dreidimensionale Gitterstrukturen bzw. gitterförmige Strukturen hergestellt werden können, die im Hinblick auf die Radialkraftverteilung, die Crimpbarkeit und die Flexibilität hervorragende Eigenschaften aufweisen.

Bei einer weiteren bevorzugten Ausführungsform wird vor dem Aufbringen der zweiten Schicht eine dritte Schicht aus einem Opfermaterial auf die zuvor in das Substrat eingedrückte Seite der Gitterstruktur aufgebracht. Dadurch werden weitere Möglichkeiten eröffnet, das Herstellungsverfahren zu variieren und somit Vorrichtungen mit unterschiedlichen Eigenschaften, beispielsweise mit Bereichen ohne bzw. mit Abdeckung herzustellen. Dabei kann die dritte Schicht aus dem Opfermaterial strukturiert werden derart, dass die Gitterstruktur teilweise mit der dritten Schicht bedeckt ist, wobei insbesondere einzelne Elemente der Gitterstruktur vollständig und/oder nicht bedeckt und/oder partiell bedeckt sind.

Das Substrat und/oder die Außenschicht können eine dreidimensionale Struktur aufweisen derart, dass die dreidimensionale Struktur auf die nachfolgend aufgebrachten Schichten übertragen wird. Dadurch ergeben sich weitere Möglichkeiten, die Eigenschaften der Vorrichtung insbesondere der Abdeckung der Vorrichtung zu variieren.

Die zweite Schicht kann beispielsweise aus bioresorbierbaren Materialien, insbesondere aus Magnesium und/oder aus röntgensichtbaren Materialien, insbesondere Tantal, hergestellt werden.

Die zweite Schicht kann die einzige Schicht zur Bildung einer einlagigen Abdeckung sein. Alternativ kann die zweite Schicht wenigstens eine weitere, insbesondere mehrere Teilschichten zur Bildung einer mehrlagigen Abdeckung umfassen. Auf diese Weise können unterschiedliche Eigenschaften der Abdeckung eingestellt werden. Beispielsweise kann eine röntgensichtbare Schicht und/oder im Fall der bioresorbierbaren Materialien Schichten mit unterschiedlichen Auflösungsgeschwindigkeiten vorgesehen sein. Zur Klarstellung wird ausgeführt, dass die Teilschichten der zweiten Schicht zur Abdeckung gehören und von der dritten Schicht zu unterscheiden sind, die eine temporär aufgebrachte Schicht aus Opfermaterial zur Strukturierung bzw. Modifizierung der Abdeckung, also der zweiten Schicht, bildet.

Nachfolgend wird die Erfindung anhand von Ausführungsbeispielen unter Bezug auf die schematischen Zeichnungen mit weiteren Einzelheiten näher erläutert. In diesem zeigen
- Fig. 1: Querschnitte durch zwei Gitterelemente mit unterschiedlichen Kanten;
- Fig. 2: den ersten Schritt der Aufbringung der Gitterstruktur auf das Substrat;
- Fig. 3: den zweiten Schritt des Eindrückens der Gitterstruktur in das Substrat;
- Fig. 4: den dritten Schritt der Beschichtung der Gitterstruktur mit einer Startschicht;
- Fig. 5: den vierten Schritt der Beschichtung der Gitterstruktur mit der ersten Schicht aus einem Opfermaterial;
- Fig. 6: den fünften Schritt der Beschichtung der Gitterstruktur mit einer zweiten Schicht zur Bildung der Abdeckung, nachdem das Substrat entfernt ist;
- Fig. 7: den sechsten Schritt der Strukturierung der zweiten Schicht zur Bildung der Abdeckung;
- Fig. 8: den siebten Schritt des Entfernens des Opfermaterials;
- Fig. 9: ein weiteres Ausführungsbeispiel des Verfahrens, bei dem eine dritte Schicht vor dem Aufbringen der zweiten Schicht aufgebracht wird;
- Fig. 10: den Schritt des Aufbringens der zweiten Schicht auf das gemäß Fig. 9 vorbehandelte Material und
- Fig. 11a-11c: ein Beispiel für ein Verfahren zur Herstellung der tragenden Gitterstruktur.

Das Verfahren zur Herstellung einer medizinischen Vorrichtung eignet sich allgemein zur Herstellung planarer Gitterstrukturen oder gitterförmiger Strukturen mit einer Abdeckung. Die planare Gitterstruktur mit der Abdeckung kann zur Bildung einer dreidimensionalen medizinischen Vorrichtung umgeformt, beispielsweise zylindrisch umgeformt werden. Durch die Umformung können Implantate, wie beispielsweise Stents, oder temporär in den Körper einführbare Geräte, wie beispielsweise Thrombosefänger oder Thrombosefilter hergestellt werden. Das Verfahren ist zur Herstellung von Gitterstrukturen mit T-förmigen Stegen, insbesondere von Stents mit derartigen Stegen geeignet. Das Verfahren ist auch zur Herstellung von Implantaten, insbesondere Stents mit vollständig abgedeckten Zellen, beispielsweise zur Herstellung eines vollständig bespannten Stents zum Stillen von Gefäßblutungen geeignet. Mit dem Verfahren können ferner Implantate, insbesondere Stents mit abgedeckten Zellen hergestellt werden, wobei die Abdeckung der Zellen eine Strukturierung, insbesondere eine Perforierung,aufweist, so dass der Stent beispielsweise als Flowdiverter eingesetzt werden kann. Das Verfahren eignet sich auch zur Herstellung von Implantaten, insbesondere von Stents mit teilweise abgedeckten Zellen, die bewegliche Klappen aufweisen, so dass die Zellen geöffnet und verschlossen werden können. Ferner können Stents mit teilweise abgedeckten Zellen zur Aneurysmenabdeckung und/oder zur Plaque-Abdeckung hergestellt werden, wobei eine Durchblutung durch Perforationen und/oder eine Durchblutung von Seitengefäßen und/oder ein Durchgang für einen Katheter zur Sondierung bzw. zum Coiling von Aneurysmen geschaffen werden können.

Zur Herstellung rotationssymmetrischer Formen, wie Stents, können die umgeformten, bspw. gerollten, ursprünglich planaren Strukturen an ihren freien Längskanten verbunden werden, insbesondere formschlüssig, kraftschlüssig oder stoffschlüssig verbunden werden, bspw. durch Verschweißen, Verkleben oder Crimpen, vorzugsweise mit Hülsen oder ähnlichen Hilfsmitteln. Danach können die Strukturen wärmebehandelt werden, um die Gefügeeigenschaften zu beeinflussen.

Es ist auch möglich, mit Hilfe des Verfahrens planare Gitterstrukturen mit einer Abdeckung herzustellen, die planar eingesetzt werden.

Das Verfahren bietet also die Möglichkeit, sowohl planare Gitterstrukturen herzustellen, die in planarer Form eingesetzt werden als auch dreidimensionale Strukturen, die aus einer planaren Anfangsstruktur mit Abdeckung gebildet werden, beispielsweise durch zylindrische Umformung. Für die dreidimensionalen Strukturen kommen beispielsweise Stents oder andere medizinische Geräte in Betracht.

Die Gitterstrukturen mit und ohne Abdeckung können auf Grund Ihrer geringen Dicke auch als Folie bezeichnet werden.

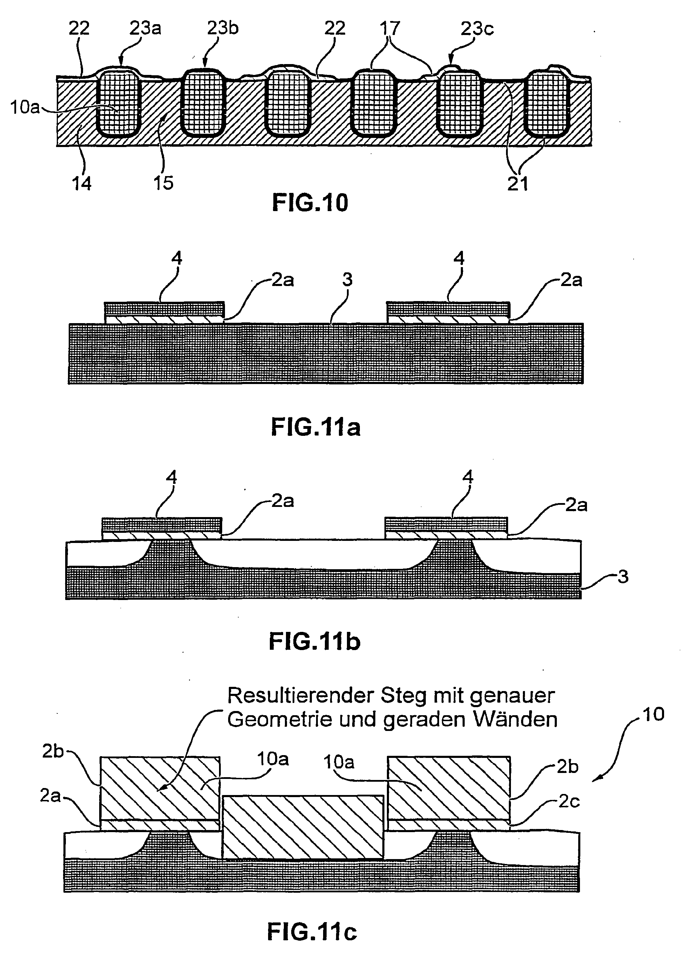

Zur Herstellung der Gitterstruktur 10 können unterschiedliche Verfahren zum Einsatz kommen. Beispielsweise ist es möglich, die Ausgangsgitterstruktur 10 durch PVD-Verfahren, insbesondere durch Magnetronsputtern herzustellen, wobei durch das Sputtern freitragende Strukturen gebildet werden. Ein Beispiel für die Herstellung der Gitterstruktur 10 durch Sputtern ist in Fig. 11a, 11b, 11c gegeben.

Dabei wird eine erste Schicht in der Form einer Stegansatzschicht 2a auf ein Substrat aufgebracht, die durch einen Ätzprozess strukturiert wird. Die stukturierte erste Schicht wird durch einen auf die Substratschicht 3 wirkenden nass-chemischen Ätzprozess unterätzt. Daraufhin wird eine Stegaufbauschicht 2b auf die erste Schicht aufgebracht. Zur Bildung der freitragenden Gitterstruktur wird die Substratschicht 3 entfernt. Bei dem Verfahren gemäß Fig. 11a bis 11c weist die Stegansatzschicht 2a eine kleinere Schichtdicke auf, als die Stegaufbauschicht 2b. Die erste Schicht mit der Stegansatzschicht 2a wird mit der Stegaufbauschicht 2b stoffschlüssig verbunden derart, dass die Stegansatzschicht 2a mit der Stegaufbauschicht 2b gemeinsam die Stege der freitragenden Gitterstruktur bilden. Das vorstehend beschriebene Verfahren zur Herstellung einer freitragenden gesputterten Gitterstruktur ist in der nicht vorveröffentlichten Anmeldung DE 10 2009 023 371 näher erläutert, die auf die Anmelderin zurückgeht und deren Inhalt in diese Anmeldung mit eingeschlossen wird. Mit diesem Verfahren zur Herstellung der tragenden Gitterstruktur 10 lassen sich sehr präzise und genaue Kanten der einzelnen Elemente der Gitterstruktur fertigen.

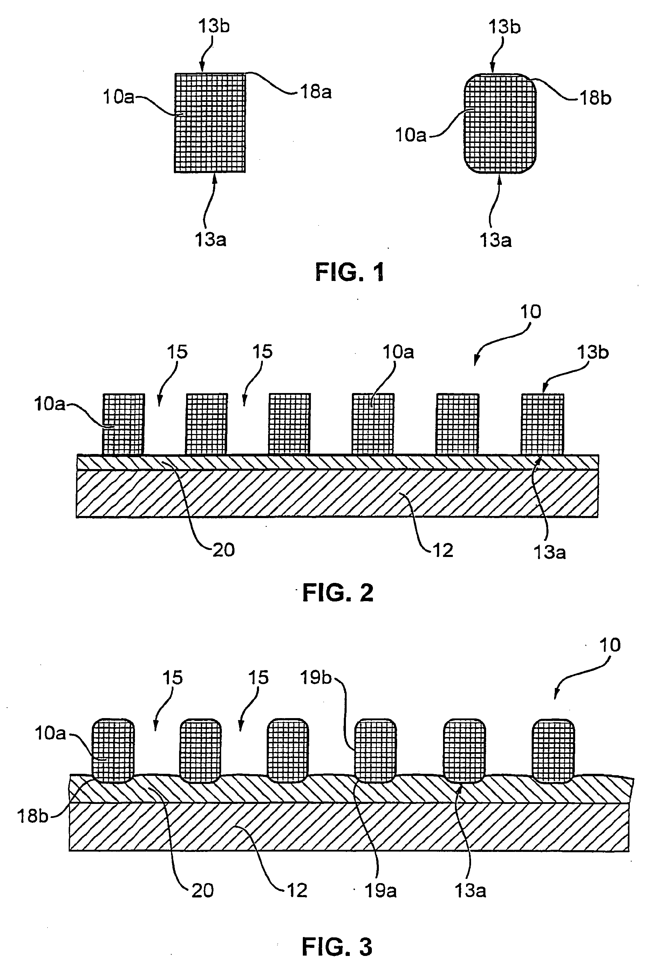

Ein Beispiel für ein Element der Gitterstruktur, die nach dem Verfahren gemäß den Fig. 11a bis 11c hergestellt ist, ist in Fig. 1 gezeigt. Darin ist zu erkennen, dass das im Querschnitt dargestellte Element 10a der Gitterstruktur 10, beispielsweise ein Steg der Gitterstruktur 10 schematisch dargestellte scharfe Kanten 18a ohne Rundung aufweist (linke Darstellung der Fig. 1). In Fig. 1, rechte Darstellung, ist der Querschnitt eines Elements 10a einer Gitterstruktur 10 dargestellt, die beispielsweise durch Laserschneiden hergestellt ist und durch Elektropolieren abgerundete Kanten 18b bzw. Abrundungen aufweist. Wie eingangs erläutert, stellen diese Abrundungen 18b bei der Verbindung des Elements 10a mit der Abdeckung ein Problem dar. Das Verfahren eignet sich nicht nur zur Verbindung des Elements 10a gemäß rechter Darstellung mit abgerundeten Kanten der Abdeckung, sondern auch zur Verbindung des Elements 10a gemäß linker Darstellung in Fig. 1.

Eine erste Seite 13a der Gitterstruktur 10 bzw. des Elements 10a ist mit einer Abdeckung zu verbinden. Die gegenüberliegende zweite Seite 13b der Gitterstruktur 10 bleibt abdeckungsfrei. Es ist auch möglich, die zweite Seite ebenfalls mit einer Abdeckung zu versehen, bspw. durch ein herkömmliches Verfahren oder durch das erfindungsgemäße Verfahren bzw. das in den Ausführungsbeispielen offenbarte Verfahren, oder anderweitig zu modifizieren. Die Zugänglichkeit der zweiten Seite für die Anbringung der Abdeckung durch das erfindungsgemäße Verfahren kann bspw. in strukturierten und/oder abdeckungsfreien Teilbereichen erfolgen.

Generell gilt, dass die Gitterstruktur der Vorrichtung auf beliebige Weise hergestellt werden kann, also beispielsweise durch Magnetronsputtern oder durch Laserschneiden, Ätzprozesse, Mikrofräseri, LIGA-Verfahren oder andere zur Herstellung von Gitterstrukturen geeignete Prozesse.

Der Schritt der Herstellung der tragenden Gitterstruktur kann Teil des gesamten Herstellungsverfahrens bilden. Es ist auch möglich, den ersten Schritt der Herstellung der Gitterstruktur separat in einem gesonderten Verfahren durchzuführen und entsprechend vorgefertigte Gitterstrukturen nach dem Verfahren gemäß dieser Anmeldung mit einer Abdeckung zu verbinden.

Die Form der Gitterstruktur kann unterschiedlich ausgebildet sein, wobei die Gitterstruktur Zellen aufweist (nicht dargestellt). Die Gitterstruktur kann offene oder geschlossene Zellen aufweisen. Die einzelnen Elemente der Gitterstruktur können Stege, Verbinder, Tips, oder andere Elemente der Gitterstruktur umfassen. Das Profil der Stege ist nicht vollständig geradlinig. Das Profil kann bauchig oder tonnenförmig sein.

Die Gitterstruktur bildet die tragende Stützstruktur der Vorrichtung. Im Fall eines Implantats, insbesondere eines Stents, bringt sie die Radialkraft auf, die auf die Gefäßwand wirkt. Die Gitterstruktur kann selbstexpandierend oder durch Aufbringen äußerer Kräfte expandierend sein (Ballondilatation). Die Materialien zur Herstellung der Gitterstruktur richten sich nach dem jeweiligen Einsatzzweck und umfassen die zur Herstellung von Implantaten oder anderen medizinischen Geräten, die temporär in den Körper eingeführt werden, üblicherweise verwendeten Materialien wie beispielsweise biokompatible Formgedächtniswerkstoffe, insbesondere NiTi -Legierungen wie Nitinol oder bioresorbierbare Materialien, wie EisenLegierungen oder Magnesium oder Tantal oder jeweils Legierungen davon oder andere Materialien.

In Fig. 2 ist dargestellt, dass bei dem ersten Arbeitsschritt die Gitterstruktur 10 auf einem Substrat 12 angeordnet wird. Das Substrat 12 ist flexibel. Dies kann bspw. dadurch erreicht werden, dass das gesamte Substrat aus einem flexiblen Material besteht. Das Substrat kann, wie bei dem dargestellten Beispiel, eine flexible Außenschicht 20 umfassen, auf der die Gitterstruktur 10 angeordnet wird. Die Außenschicht 20 kann beispielsweise ein Fotolack sein. Das Substrat kann in diesem Fall einen harten Tragabschnitt umfassen, auf dem die Außenschicht20 aufgebracht ist. Vorteilhafterweise ist die Außenschicht 20 plastisch verformbar.

Durch das geringe Eigengewicht der Gitterstruktur 10, bzw. Folie erfolgt bei dem ersten Arbeitsschritt keinerlei Formänderung des Substrates 12, bzw. der Außenschicht 20. Außerdem ist die Gitterstruktur 10, bzw. Folie prozessbedingt leicht gekrümmt, so dass Bereiche der Gitterstruktur 10 das Substrat 12 nicht berühren. Ohne äußere Krafteinwirkung ist ein Eindrücken der Gitterstruktur 10 nicht möglich.

Die Gitterstruktur 10 wird mit einer ersten Seiten 13a bzw. derjenigen Seite 13a, die mit der Abdeckung zu verbinden ist, auf dem Substrat 12, insbesondere auf dem Fotolack 20 positioniert. Der Fotolack bewirkt durch seine Hafteigenschaften, dass die Gitterstruktur 10 auf dem Substrat ausreichend gut fixiert ist. Die Zwischenräume 15 zwischen den einzelnen Gitterelementen 10a bzw. die Öffnungen der Gitterstruktur 10 erstrecken sich im Wesentlichen senkrecht zur Oberfläche des Substrats 12. Das Substrat 12 ist durch die Zwischenräume 15 hindurch zugänglich.

An dieser Stelle wird darauf hingewiesen, dass der Begriff Substrat sowohl den tragenden Teil als auch den flexiblen Teil umfasst, auf dem die Gitterstruktur 10 angeordnet wird. Es ist möglich, das Substrat insgesamt aus einem flexiblen Material herzustellen, das ausreichend plastisch verformbar ist, um das Eindrücken der Gitterstruktur zu ermöglichen. Das Substrat kann also einteilig oder zweiteilig, insbesondere mehrteilig bzw. mehrschichtig aufgebaut sein.

In den Fig. 2 und 3 sind einmal Gitterelemente 10a mit präzisen Kanten (Fig. 2) und Gitterelemente 10a mit abgerundeten Kanten (Fig. 3) dargestellt. Das Verfahren ist zur Verbindung der Abdeckung mit der Gitterstruktur 10 in beiden Fällen geeignet.

Im zweiten Arbeitsschritt gemäß Fig. 3 wird die Gitterstruktur 10 in das Substrat 12, insbesondere in die Außenschicht 20 des Substrates 12 eingedrückt und zwar aktiv, also durch eine äußere Krafteinwirkung eingedrückt. Dazu wird eine äußere Kraft auf die Gitterstruktur 10 aufgebracht, die im Wesentlichen senkrecht zum Substrat wirkt. Die äußere Kraft ist so stark, dass die Gitterstruktur 10 zumindest im Bereich der Kanten 18a bzw. der Abrundungen 18b auf der ersten Seite 13a der Gitterstruktur in das Substrat 12 bzw. die Außenschicht 20 eingedrückt wird. Die Eindrücktiefe kann in den eingangs erwähnten Bereichsgrenzen variiert werden, die ggf. noch über- oder unterschritten werden können. Die für das Eindrücken erforderliche Kraft wird vom Fachmann beispielsweise experimentell bestimmt. Der für das Eindrücken erforderliche Druck ist flächenabhängig. Die Kraft kann bspw. zwischen 0,5 bis 5 N liegen.

Das Eindrücken der Gitterstruktur 10 erfolgt flächig. Überdies erfolgt das Eindrücken der Gitterstruktur 10 homogen, so dass eine möglichst gleichmäßige Eindrücktiefe über die flächige Erstreckung der Gitterstruktur 10 erreicht wird. Wenn die Kraft zum Eindrücken aufgebracht wird, liegt die Gitterstruktur 10 plan auf dem Substrat 12 auf, so dass alle Elemente 11 der Gitterstruktur 10, bzw. im Wesentlichen alle Elemente 11 der Gitterstruktur 10 das Substrat berühren. Durch den auf die Gitterstruktur 10 wirkenden äußeren Druck wird bewirkt, dass sich die Oberfläche des Substrats 12 bzw. der Außenschicht 20 verformt, insbesondere plastisch verformt. Es wird dabei ein Profil in der Oberfläche gebildet, das der Gitterstruktur 10, insbesondere der Anordnung der Elemente 11 der Gitterstruktur 10 entspricht. Die Elemente 11 dringen dadurch in die ursprüngliche plane Oberfläche des Substrats 12 bzw. der Außenschicht 20 und bilden Vertiefungen in der Oberfläche des Substrats 12, bzw. der Außenschicht 20, in denen die Elemente 11 teilweise angeordnet bzw. aufgenommen sind. Im Hinblick auf die Zahlenwerte der Eindrücktiefe, die der Dimension der Vertiefungen entspricht, wird auf die eingangs genannten Bereiche einschließlich der genannten Zwischenwerte bzw. Bereichsgrenzen Bezug genommen.

Für das flächige und homogene Eindrücken der Gitterstruktur 10 ist eine Platte mit einer hochgenauen Oberfläche vorgesehen, die auf der der Substrat 10 abgewandten Seite 13b der Gitterstruktur 10 angeordnet ist und durch ein äußere Krafteinwirkung auf die Gitterstruktur 10 gedrückt wird. Hierzu sind an sich bekannte Stellmittel vorgesehen, die Stellweglängen im erforderlichen nm-Bereich oder µm-Bereich ermöglichen. Zur Messung der Eindrücktiefe kann bspw. ein an sich bekanntes Profilometer verwendet werden, das das Profil der Gitterstruktur 10 und des Substrates 12 mit Nanometer-Genauigkeit abfährt.

Wie in Fig. 3 dargestellt, wird die Gitterstruktur 10 teilweise in die Außenschicht 20 eingedrückt. Die in Fig. 3 gezeigte Eindrücktiefe bzw. Eindringtiefe der Gitterstruktur 10 in das Substrat ist beispielhaft. Es ist auch möglich, die Gitterstruktur 10 stärker in die Außenschicht einzudrücken, beispielsweise um einen höheren Abdeckungsgrad der Kantenbereiche der Gitterstruktur 10 zu erreichen. Die Gitterstruktur 10 wird so tief in das Substrat 12 bzw. die Außenschicht 20 eingedrückt, dass die Kanten 18a bzw. die Abrundungen 18b zumindest teilweise in der Außenschicht 20 bzw. im Substrat verschwinden bzw. darin eingebettet werden. Damit wird erreicht, dass die Elemente 10a im Bereich der Kanten im Wesentlichen vollständig vom Substrat 12 bzw. der Außenschicht 20 berührt bzw. bedeckt werden, selbst wenn die Wandstärke der Elemente 10a schwankt.

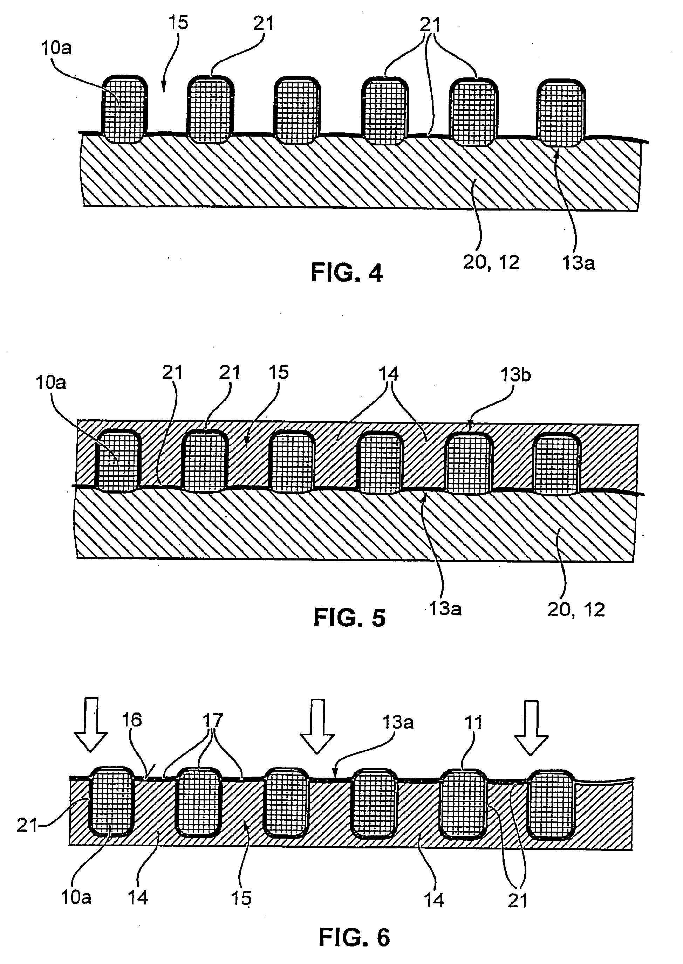

Im dritten Arbeitsschritt gemäß Fig. 4 wird ein Zwischenmaterial bzw. eine Startschicht 21 auf die Gitterstruktur 10 und das Substrat 12 bzw. die Außenschicht 20 aufgebracht. Wie in Fig. 4 zu erkennen, wird durch die Beschichtung der Gitterstruktur 10 mit der Startschicht 21 auch das Substrat 12 bzw. die Außenschicht 20 beschichtet und zwar im Bereich der Zwischenräume 15, so dass sich eine vollständige Abdeckung oder zumindest eine im Wesentlichen vollständige Abdeckung der einzelnen Gitterelemente 10a ergibt, bis auf denjenigen Bereich der Gitterelemente 10a, der im Substrat 12 bzw. in der Außenschicht 20 eingebettet ist bzw. hineingedrückt ist. Durch den vollständigen Kontakt der Elemente 10a bzw. der Stege bzw. durch das Hineindrücken der Elemente 10a bzw. der Stege in das Substrat 12 werden Schattenstellen vermieden, in denen das Zwischenmaterial keinen Kontakt mit den Elementen 10a bzw. Stegen aufweisen würde.

Ein geeignetes Material für die Startschicht 21 bildet beispielsweise Kupfer, das auf die Gitterstruktur 10a gesputtert wird.

In Fig. 4 ist die Unterscheidung zwischen Außenschicht 20 und tragendem Substrat 12 nicht dargestellt. Fig. 4 ist, ebenso wie die anderen Figuren, so zu verstehen, dass die Gitterstruktur 10 entweder in die Außenschicht 20, beispielsweise den Fotolack oder in die tragende Struktur des Substrats eingedrückt wird, die sich jeweils plastisch verformen.

Im vierten Arbeitsschritte gemäß Fig. 5 ist dargestellt, dass die Zwischenräume 15 bzw. die Öffnungen der Gitterstruktur 10 durch eine erste Schicht 14 eines Opfermaterials aufgefüllt werden. Vorzugsweise werden die Zwischenräume 15 vollständig mit der ersten Schicht 14 aus dem Opfermaterial aufgefüllt, so dass sich insgesamt eine stabile und leicht zu handhabende Einheit aus Gitterstruktur 10 und erster Schicht 14 ergibt. Vorzugsweise bedeckt die erste Schicht 14 die von der ersten Seite 13a der Gitterstruktur abgewandte zweite Seite 13b, so dass ein besonders fester und haltbarer Kontakt zwischen der ersten Schicht 14 und der Gitterstruktur 10 erreicht wird. Es ist auch möglich, die Zwischenräume 15 nur teilweise, beispielsweise nur bis zur Hälfte der Höhe der Elemente 10a zu füllen. Es ist ausreichend, dass die Zwischenräume 15 der Gitterstruktur 10 mit der ersten Schicht 14 so bedeckt werden, dass die Gitterstruktur 10 zusammen mit der ersten Schicht 14 auf der mit der Abdeckung zu verbindenden ersten Seite 13a eine im Wesentlichen geschlossene Fläche 16 bildet, nachdem das Substrat entfernt wird (Fig. 6). Die geschlossene Fläche 16 bildet eine im Wesentlichen durchgängige Kontaktfläche für die nachfolgend abzuscheidende zweite Schicht 17 zur Bildung der Abdeckung 11 (Fig. 6).

Die erste Schicht 14 aus dem Opfermaterial wird durch einen galvanischen Prozess aufgewachsen. Vorteilhafterweise wird die erste Schicht 14 zum Befüllen der Zwischenräume 15 aus demselben Material hergestellt, wie die Startschicht 21. Es ist auch möglich nur Teilbereiche der Gitterstruktur mit der Opferschicht zu befüllen.

Im fünften Arbeitsschritt gemäß Fig. 6 wird die Strukturabdeckung gebildet. Dazu wird durch ein Dünnschichtverfahren eine Folie bzw. eine zweite Schicht 17 zur Bildung der Abdeckung auf die zuvor in das Substrat 12 bzw. die Außenschicht 20 eingedrückte Seite 13a der Gitterstruktur 10 aufgebracht, nachdem das Substrat 12 einschließlich der Außenschicht 20 entfernt wurde (Fig. 6). Die zweite Schicht 17 kann durch ein PVD-Verfahren, insbesondere durch Magnetronsputtern oder ein anderes Verfahren aufgetragen werden. Wie in Fig. 6 gut zu erkennen, bedeckt die zweite Schicht 17 die über die erste Schicht 14 aus dem Opfermaterial hervorstehenden Bereiche der Elemente 10a der Gitterstruktur 10. Konkret werden die zuvor in das Substrat 12 bzw. die Außenschicht 20 eingedrückten Bereiche der Elemente 10, d.h. die kritischen Kantenbereiche der Elemente 10, die nach Entfernen des Substrats 12 bzw. der Außenschicht 20 freiliegen, durch die Schicht 17 bedeckt. Dadurch wird eine besonders gute Anhaftung der Schicht 17 bzw. allgemein der Folie an den Elementen 10, 10a bzw. an den Stegen erreicht. Das Aufbringen der zweiten Schicht 17 auf der zuvor vom Substrat 12 bedeckten Seite 13a der Gitterstruktur 10 wird durch drei senkrecht zur Gitterstruktur 10 verlaufende Pfeile in Fig. 6 dargestellt.

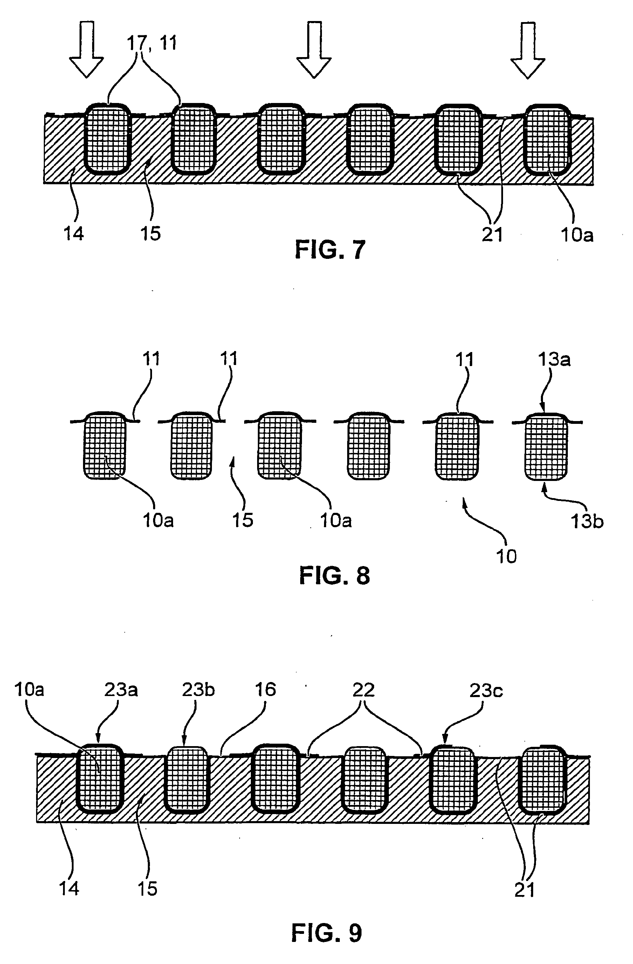

Im sechsten Arbeitsschritt gemäß Fig. 7 wird die durchgängige Schicht 17 bzw. Folie strukturiert, beispielsweise durch ein an sich bekanntes lithografisches Verfahren. Dadurch wird die Form der Abdeckung, beispielsweise, wie in Fig. 7 dargestellt, die im Zusammenhang mit den Elementen 10a sich bildende T-förmige Querschnittsform hergestellt.

Im siebten Arbeitsschritt gemäß Fig. 8 wird die erste Schicht 14 aus dem Opfermaterial einschließlich etwaiger Startschichten 21 entfernt, beispielsweise durch Ätzen. Somit verbleiben die Elemente 10a bzw. die Gitterstruktur 10 mit der Abdeckung 11 als End- bzw. möglicherweise auch als Zwischenprodukt, das einer weiteren Behandlung unterzogen wird. Beispielsweise kann die Gitterstruktur 10 mit der Abdeckung 11 einer Wärmebehandlung unterzogen werden, um im Fall von Formgedächtnisschichten, beispielsweise aus NiTi-Legierungen, das Gefüge auf eine Umwandlungstemperatur einzustellen. Dadurch können auch dreidimensionale Formen der Gitterstruktur, bspw. zylindrische Formen (Stents) fixiert werden.

Mit dem Verfahren können die Gitterstruktur 10 und die Abdeckung 11 aus demselben Material, beispielsweise aus einem Formgedächtnismaterial hergestellt sein. Andere Materialien, wie beispielsweise bioresorbierbare Materialien wie beispielsweise Magnesium oder Eisen sind ebenfalls möglich. Es ist auch möglich, mit dem Verfahren unterschiedliche Materialien für die Gitterstruktur 10 und die Abdeckung 11 zu kombinieren. Beispielsweise kann das Gerüst bzw. die Gitterstruktur aus einem Formgedächtniswerkstoff, beispielsweise aus einer NiTi-Legierung und die umhüllende Schicht bzw. die Abdeckung 11 aus Eisen hergestellt sein. Ferner ist es auch möglich, die Abdeckung 11 bzw. allgemein die umhüllende äußere Schicht aus einem bioresorbierbaren Material, beispielsweise aus Polymeren herzustellen. Es ist auch möglich, umgekehrt die Gitterstruktur 10 aus einem bioresorbierbaren Material und die Abdeckung 11 aus einem beständigen Material herzustellen.

In den Fig. 9 und 10 ist eine Alternative bzw. Erweiterung des Verfahrens gemäß den Fig. 2 bis 8 dargestellt. Die Arbeitsschritte gemäß den Fig. 2 bis 5 einschließlich des Schrittes des Entfernens des Substrats 12 können dem Arbeitsschritt gemäß Fig. 9 vorausgehen.

Nach dem Entfernen des Substrats 12 bzw. der Außenschicht 20 bzw. allgemein nach dem Freilegen der mit der Abdeckung 11 zu verbindenden Seite 13a der Gitterstruktur 10 wird eine dritte Schicht 22 aus einem Opfermaterial auf die erste Seite 13a aufgebracht. Die dritte Schicht 22 kann strukturiert werden. Die Strukturierung kann so erfolgen, dass die Elemente 10a nur teilweise bedeckt bleiben (siehe partielle Überdeckung 23c). Es ist auch möglich, die Elemente 10a vollständig zu überdecken (siehe vollständige Überdeckung 23a) oder einige Elemente 10a freizulassen (siehe ohne Überdeckung 23b). Die vorstehend genannten Strukturierungsmöglichkeiten können beliebig kombiniert werden.

Im darauffolgenden Arbeitsschritt gemäß Fig. 10 wird die zweite Schicht 17 zur Bildung der Abdeckung aufgebracht. Zur Klarstellung wird ausgeführt, dass die dritte Schicht 22 als Opferschicht zeitlich vor der zweiten Schicht 17 aufgebracht wird. Durch die partielle Überdeckung 23c bzw. die vollständige Überdeckung 23a erfolgt in den überdeckten Bereichen keine Verbindung zwischen der zweiten Schicht 17 und der Oberfläche der Elemente 10a. Die Verbindung zwischen der zweiten Schicht 17 und der Oberfläche der Elemente 10a erfolgt nur in den unbedeckten Bereichen, also in den vollständig unbedeckten Bereichen 23b bzw. in den partiell unbedeckten Bereichen 23c. Durch die teilweise Verbindung der Folie bzw. Schicht 17 bzw. Abdeckung 11 wird eine Modifizierung der Abdeckung 11 erreicht. Bspw. können die Abdeckung und die Gitterstruktur bereichsweise relativ beweglich zueinander angeordnet sein. Die Abdeckung kann in der Form von klappen ausgeführt sein. Durch die Relativbewegung zwischen Abdeckung und Gitterstruktur wird eine erhöhte Flexibilität beim Crimpen erreicht.

Die dritte Schicht 22 aus dem Opfermaterial wird vor der endgültigen Herstellung der Vorrichtung entfernt.

Bei einem weiteren Ausführungsbeispiel kann das Substrat 12 und/oder die Außenschicht 20 eine dreidimensionale Struktur aufweisen. Beispielsweise können Rillen und/oder Vorsprünge in der Oberfläche des Substrats 12 bzw. der Außenschicht 20 ausgebildet sein. Beim nachfolgenden Aufsputtern der zweiten Schicht 17 zur Bildung der Abdeckung 11 wird die dreidimensionale Struktur des Substrats 12 bzw. der Außenschicht 20 auf die zweite Schicht 17 bzw. die Abdeckung 11 übertragen. Dies hat den Vorteil, dass dreidimensionale Strukturen für die Optimierung beispielsweise der Strömungsverhältnisse und/oder der Endothelialisierung aufgebaut werden können. Alternativ oder zusätzlich können Faltlinien in der Abdeckung 11 gebildet werden, die ein Crimpen des Gesamtsystems zum Einführen in einen Katheter bzw. allgemein in ein Zufuhrsystem erleichtern.

Bei einem weiteren bevorzugten Ausführungsbeispiel können bioresorbierbare Materialien, wie beispielsweise Magnesium oder Eisen und/oder röntgensichtbare Materialien wie beispielsweise Tantal zur Bildung der Abdeckung 11 aufgebracht werden. Dabei ist es möglich, dass die Abdeckung 11 mehrlagig ausgebildet ist und mehrere Schicht aus unterschiedlichen Materialien aufweist. Alternativ ist es möglich, die Abdeckung 11 aus einer einzigen Schicht bzw. aus mehreren Teilschichten aus dem selben Material einlagig herzustellen. Die Schicht kann aus einem bioresorbierbaren oder aus einem röntgensichtbaren Material bestehen.

Das Aufbringen der zweiten Schicht 17 bzw. generell der Schicht zur Bildung der Abdeckung 11 durch ein Dünnschichtverfahren, insbesondere durch Sputtern bzw. Magnetronsputtern hat den Vorteil, dass eine sehr dünne Folie aufgebracht werden kann, die insbesondere dünner ist, als die durch herkömmliche Verfahren mit der Stützstruktur verbundene vorgefertigte Folie. Die Verbindung sehr dünner Folien durch herkömmliche Verfahren führt häufig zu Handhabungsproblemen beim Herstellungsprozess. Außerdem kann es bei der Verbindung zu Fehlern kommen, die die Stabilität der gesamten Struktur gefährden. Beim herkömmlichen Schweißen dünner Folien muss die für das Schweißen erforderliche Energie genau reguliert werden. Trotzdem ist die Gefahr der Materialverbrennung sehr hoch. Beim Kleben sehr dünner Strukturen kommt es zu Kapillareffekten, wodurch der Kleber in unerwünschte Bereiche der Struktur vordringt.

Demgegenüber hat die Herstellung der Abdeckung 11 durch eine Dünnschichttechnologie, insbesondere durch Sputtern den Vorteil, dass die Abdeckung 11 bei der Herstellung in situ mit der Gitterstruktur 10 verbunden wird. Auf diese Weise können sehr dünne Folien auf der Gitterstruktur 10 aufgebracht werden, ohne dass es zu größeren Handhabungsproblemen bei der Herstellung kommt. Diese dünnen Folien lassen sich leichter crimpen, um in einen Katheter eingebracht zu werden.

### Bezugszeichenliste

- 10: Gitterstruktur
- 10a: Elemente der Gitterstruktur
- 11: Abdeckung
- 12: Substrat
- 13a: erste Seite
- 13b: zweite Seite
- 14: erste Schicht
- 15: Zwischenräume
- 16: Fläche
- 17: zweite Schicht
- 18a: Kanten
- 18b: Abrundungen
- 19a: erster Teil
- 19b: zweiter Teil
- 20: Außenschicht/Fotolack
- 21: Startschicht
- 22: dritte Schicht
- 23a: vollständige Überdeckung
- 23b: ohne Überdeckung
- 23c: partielle Überdeckung

## Patentansprüche

1. Verfahren zur Herstellung einer medizinischen Vorrichtung mit einer Gitterstruktur (10), die zumindest abschnittsweise mit einer Abdeckung (11) verbunden ist, bei dem
- die Gitterstruktur (10) mit der Seite (13a), die mit der Abdeckung (11) zu verbinden ist, auf einem Substrat (12) angeordnet wird,
- die Gitterstruktur (10) mit der auf dem Substrat (12) angeordneten Seite (13a) in das Substrat (12) zumindest teilweise eingedrückt wird,
- die Gitterstruktur (10) mit einer ersten Schicht (14) aus einem Opfermaterial beschichtet wird, so dass zumindest ein Teil der Zwischenräume (15) der Gitterstruktur (10) mit der ersten Schicht (14) bedeckt wird und die erste Schicht (14) mit der Gitterstruktur (10) auf der mit der Abdeckung (11) zu verbindenden Seite (13a) eine im Wesentlichen geschlossene Fläche (16) bildet,
- das Substrat (12) entfernt wird, so dass die Seite (13a) der Gitterstruktur (10) zugänglich ist, die mit der Abdeckung (11) zu verbinden ist,
- eine zweite Schicht (17) zur Bildung der Abdeckung (11) auf die zuvor in das Substrat (12) eingedrückte Seite (13a) der Gitterstruktur (10) aufgebracht wird und
- das Opfermaterial entfernt wird.

2. Verfahren nach Anspruch 1
**dadurch gekennzeichnet,dass**
die Gitterstruktur (10) derart in das Substrat (12) eingedrückt wird, dass Kanten (18a) oder seitliche Abrundungen (18b) der Gitterstruktur (10) im Substrat (12) eingebettet sind.

3. Verfahren nach Anspruch 1 oder 2
**dadurch gekennzeichnet, dass**
das Verhältnis zwischen dem in das Substrat (12) eingedrückten Teil (19a) der Gitterstruktur (10) und dem außerhalb des Substrats (12) befindlichen Teil (19b) der Gitterstruktur (10) mindestens 0,0005, insbesondere mindestens 0,001, insbesondere mindestens 0,005, insbesondere mindestens 0,01, insbesondere mindestens 0,03, insbesondere mindestens 0,05, insbesondere mindestens 0,07, insbesondere mindestens 0,1, insbesondere mindestens 0,15, insbesondere mindestens 0,2 beträgt.

4. Verfahren nach wenigstens einem der vorhergehenden Ansprüche
**dadurch gekennzeichnet, dass**
das Substrat (12) flexibel ist und/oder eine Außenschicht (20) aus einem flexiblen Material, insbesondere eine Außenschicht (20) aus Fotolack oder einem viskosen Polymer aufweist, die beim Eindrücken des Substrats (12) nachgibt.

5. Verfahren nach Anspruch 4
**dadurch gekennzeichnet,dass**
zum Entfernen des Substrats (12) von der Gitterstruktur (10) die Außenschicht (20) des Substrats (12) entfernt wird.

6. Verfahren nach wenigstens einem der vorhergehenden Ansprüche
**dadurch gekennzeichnet,dass**
die Gitterstruktur (10) vor dem Beschichten mit der ersten Schicht (14) aus einem Opfermaterial mit einer Startschicht (21) beschichtet wird.

7. Verfahren nach wenigstens einem der vorhergehenden Ansprüche
**dadurch gekennzeichnet, dass**
das Opfermaterial der ersten Schicht (14) und/oder die Startschicht (21) aus einem metallischen Material, insbesondere aus demselben metallischen Material oder aus unterschiedlichen metallischen Materialien gebildet werden.

8. Verfahren nach wenigstens einem der vorhergehenden Ansprüche
**dadurch gekennzeichnet, dass**
das Beschichten der Gitterstruktur (10) mit der Startschicht (21) durch ein Gasphasenabscheidungsverfahren, insbesondere ein PVD-Verfahren, insbesondere durch Sputtern und/oder das Beschichten der Gitterstruktur (10) mit der ersten Schicht (14) aus einem Opfermaterial durch ein galvanisches Verfahren erfolgt.

9. Verfahren nach wenigstens einem der vorhergehenden Ansprüche
**dadurch gekennzeichnet, dass**
beim Beschichten der Gitterstruktur (10) mit der ersten Schicht (14) aus einem Opfermaterial die Gitterstruktur (10) teilweise oder vollständig in das Opfermaterial eingebettet wird.

10. Verfahren nach wenigstens einem der vorhergehenden Ansprüche
**dadurch gekennzeichnet, dass**
die zweite Schicht (17) zur Bildung der Abdeckung (11) durch ein Gasphasenabscheidungsverfahren, insbesondere ein PVD-Verfahren, insbesondere durch Sputtern aufgebracht wird.

11. Verfahren nach wenigstens einem der vorhergehenden Ansprüche
**dadurch gekennzeichnet, dass**
die zweite Schicht (17) zur Bildung der Abdeckung (11) nach dem Aufbringen strukturiert wird, insbesondere durch ein lithografisches Verfahren.

12. Verfahren nach wenigstens einem der vorhergehenden Ansprüche
**dadurch gekennzeichnet,dass**
vor dem Aufbringen der zweiten Schicht (17) eine dritte Schicht (22) aus einem Opfermaterial auf die zuvor in das Substrat (12) eingedrückte Seite (13a) der Gitterstruktur (10) aufgebracht wird.

13. Verfahren nach Anspruch 12
**dadurch gekennzeichnet, dass**
die dritte Schicht (22) aus einem Opfermaterial strukturiert wird derart, dass die Gitterstruktur (10) teilweise mit der dritten Schicht (22) bedeckt ist, wobei insbesondere einzelne Elemente der Gitterstruktur (10) vollständig und/oder nicht bedeckt und/oder partiell bedeckt sind.

14. Verfahren nach wenigstens einem der vorhergehenden Ansprüche
**dadurch gekennzeichnet, dass**
das Substrat (12) und/oder die Außenschicht (20) eine dreidimensionale Struktur aufweisen derart, dass die Struktur auf die nachfolgend aufgebrachten Schichten übertragen wird.

15. Verfahren nach wenigstens einem der vorhergehenden Ansprüche
**dadurch gekennzeichnet, dass**
zum Aufbringen der zweiten Schicht (17) bioresorbierbare Materialien, insbesondere Magnesium, und/oder röntgensichtbare Materialien, insbesondere Tantal oder jeweils Legierungen davon verwendet werden.

16. Verfahren nach wenigstens einem der vorhergehenden Ansprüche
**dadurch gekennzeichnet, dass**
die zweite Schicht (17) die einzige Schicht zur Bildung einer einlagigen Abdeckung (11) ist oder auf die zweite Schicht (17) wenigstens eine weitere, insbesondere mehrere Schichten zur Bildung einer mehrlagigen Abdeckung (11) aufgebracht werden.

## Claims

1. A method for manufacturing a medical device having a grid structure (10), which is connected to a cover (11) in at least some sections, wherein
- the grid structure (10) is disposed on a substrate (12) with the side (13a) that is to be connected to the cover (11),
- the grid structure (10) is at least partially pressed into the substrate (12) on the side (13a) disposed on the substrate (12),
- the grid structure (10) is coated with a first layer (14) of a sacrificial material, so that at least some of the interspaces (15) of the grid structure (10) are covered with the first layer (14), and the first layer (14) forms an essentially closed surface (16) with the grid structure (10) on the side (13a) to be connected to the cover (11),
- the substrate (12) is removed, so that the side (13a) of the grid structure (10) that is to be connected to the cover (11) is accessible,
- a second layer (17) for forming the cover (11) is applied to the side (13a) of the grid structure (10) that was previously pressed into the substrate (12), and
- the sacrificial material is removed.

2. The method according to Claim 1, **characterized in that** the grid structure (10) is pressed into the substrate (12) in such a way that edges (18a) or rounded lateral shapes (18b) of the grid structure (10) are embedded in the substrate (12).

3. The method according to Claim 1 or 2, **characterized in that** the ratio between the part (19a) of the grid structure (10) pressed into the substrate (12) and the part (19b) of the grid structure (10) outside of the substrate (12) amounts to at least 0.0005, in particular at least 0.001, in particular at least 0.005, in particular at least 0.01, in particular at least 0.03, in particular at least 0.05, in particular at least 0.07, in particular at least 0.1, in particular at least 0.15, in particular at least 0.2.

4. The method according to at least one of the preceding claims, **characterized in that** the substrate (12) is flexible and/or has an outer layer (20) made of a flexible material, in particular an outer layer (20) of a photoresist or a viscous polymer, which yields when pressed into the substrate (12).

5. The method according to Claim 4, **characterized in that** to remove the substrate (12) from the grid structure (10), the outer layer (20) of the substrate (12) is removed.

6. The method according to at least one of the preceding claims, **characterized in that** the grid structure (10) is coated with a starting layer (21) before coating with the first layer (14) of a sacrificial material.

7. The method according to at least one of the preceding claims, **characterized in that** the sacrificial material of the first layer (14) and/or the starting layer (21) is/are formed from a metallic material, in particular from the same metallic material or from different metallic materials.

8. The method according to at least one of the preceding claims, **characterized in that** the coating of the grid structure (10) with the starting layer (21) is performed by a gas-phase deposition method, in particular a PVD method, in particular by sputtering and/or coating the grid structure (10) with the first layer (14) of a sacrificial material by a galvanic method.

9. The method according to at least one of the preceding claims, **characterized in that** in coating the grid structure (10) with the first layer (14) of a sacrificial material, the grid structure (10) is partially or completely embedded in the sacrificial material.

10. The method according to at least one of the preceding claims, **characterized in that** the second layer (17) is applied by a gas-phase deposition method, in particular a PVD method, in particular by sputtering, to form the cover (11).

11. The method according to at least one of the preceding claims, **characterized in that** the second layer (17) to form the cover (11) is structured after being applied, in particular by a lithographic process.

12. The method according to at least one of the preceding claims, **characterized in that** before applying the second layer (17), a third layer (22) of a sacrificial material is applied to the side (13a) of the grid structure (10) that was previously pressed into the substrate (12).

13. Method according to Claim 12, **characterized in that** the third layer (22) is structured from a sacrificial material, such that the grid structure (10) is partially covered with the third layer (22), wherein individual elements of the grid structure (10) in particular are covered completely and/or partially and/or not at all.

14. The method according to at least one of the preceding claims, **characterized in that** the substrate (12) and/or the outer layer (20) has/have a three-dimensional structure such that the structure is transferred to the layers applied subsequently.

15. The method according to at least one of the preceding claims, **characterized in that** for applying the second layer (17), bioabsorbable materials, in particular magnesium and/or X-ray visible materials, in particular tantalum or alloys thereof are used.

16. The method according to at least one of the preceding claims, **characterized in that** the second layer (17) is the only layer to form a single-layer cover (11), or at least one additional layer, in particular multiple layers, are applied to the second layer (17) to form a multilayer cover (11).

## Revendications

1. Procédé de fabrication d'un dispositif médical avec une structure grillagée (10) qui est reliée du moins par sections à un recouvrement (11), dans lequel
- la structure grillagée (10) est disposée sur un substrat (12) par sa face (13a) qui doit être reliée au recouvrement (11),
- la structure grillagée (10) est enfoncée du moins partiellement dans le substrat (12) par sa face (13a) disposée sur le substrat (12),
- la structure grillagée (10) est enrobée d'une première couche (14) de matériau sacrifié, de sorte qu'au moins une partie des intervalles (15) de la structure grillagée (10) est recouverte par la première couche (14) et que la première couche (14) constitue avec la structure grillagée (10) sur la face (13a) à relier au recouvrement (11) une surface sensiblement fermée (16),
- le substrat (12) est enlevé pour que la face (13a) de la structure grillagée (10) qui doit être reliée au recouvrement (11) soit accessible,
- une seconde couche (17) est appliquée pour former le recouvrement (11) sur la face (13a) préalablement enfoncée dans le substrat (12) de la structure grillagée (10) et
- le matériau sacrifié est enlevé.

2. Procédé selon la revendication 1, **caractérisé en ce que** la structure grillagée (10) est enfoncée dans le substrat (12) de manière à ce que les bords (18a) ou les arrondis latéraux (18b) de la structure grillagée (10) soient encastrés dans le substrat (12).

3. Procédé selon la revendication 1 ou 2, **caractérisé en ce que** le rapport entre la partie (19a) enfoncée dans le substrat (12) de la structure grillagée (10) et la partie (19b) se trouvant en dehors du substrat (12) de la structure grillagée (10) s'élève à au moins 0,0005, en particulier au moins 0,001, en particulier au moins 0,005, en particulier au moins 0,01, en particulier au moins 0,03, en particulier au moins 0,05, en particulier au moins 0,07, en particulier au moins 0,1, en particulier au moins 0,15, en particulier au moins 0,2.

4. Procédé selon au moins une des revendications précédentes, **caractérisé en ce que** le substrat (12) est souple et/ou comporte une couche extérieure (20) en matériau souple, en particulier une couche extérieure (20) en vernis photosensible ou en polymère visqueux qui cède lors de l'enfoncement du substrat (12).

5. Procédé selon la revendication 4, **caractérisé en ce que**, pour enlever le substrat (12) de la structure grillagée (10), on enlève la couche extérieure (20) du substrat (12).

6. Procédé selon au moins une des revendications précédentes, **caractérisé en ce que** la structure grillagée (10) est enrobée d'une couche initiale (21) en matériau sacrifié avant d'être enrobée avec la première couche (14).

7. Procédé selon au moins une des revendications précédentes, **caractérisé en ce que** le matériau sacrifié de la première couche (14) et/ou la couche initiale (21) sont constitués d'un matériau métallique, en particulier du même matériau métallique ou de différents matériaux métalliques.

8. Procédé selon au moins une des revendications précédentes, **caractérisé en ce que** l'enrobage de la structure grillagée (10) avec la couche initiale (21) se fait par un procédé de séparation en phase gazeuse, en particulier un procédé de PVD, en particulier par pulvérisation cathodique et/ou enrobage de la structure grillagée (10) avec la première couche (14) en matériau sacrifié par procédé de galvanisation.

9. Procédé selon au moins une des revendications précédentes, **caractérisé en ce que** lors de l'enrobage de la structure grillagée (10) avec la première couche (14) en matériau sacrifié, la structure grillagée (10) est encastrée partiellement ou complètement dans le matériau sacrifié.

10. Procédé selon au moins une des revendications précédentes, **caractérisé en ce que** la seconde couche (17) permettant de constituer le recouvrement (11) est appliquée par procédé de séparation en phase gazeuse, en particulier procédé de PVD, en particulier par pulvérisation cathodique.

11. Procédé selon au moins une des revendications précédentes, **caractérisé en ce que** la seconde couche (17) permettant de constituer le recouvrement (11) est structurée après application, en particulier par procédé lithographique.

12. Procédé selon au moins une des revendications précédentes, **caractérisé en ce que** avant d'appliquer la seconde couche (17), une troisième couche (22) en matériau sacrifié est appliqué sur la face (13a) préalablement enfoncée dans le substrat (12) de la structure grillagée (10).

13. Procédé selon la revendication 12, **caractérisé en ce que** la troisième couche (22) en matériau sacrifié est structurée de manière à ce que la structure grillagée (10) soit recouverte partiellement par la troisième couche (22), des éléments individuels de la structure grillagée (10) étant en particulier recouverts complètement et/ou non recouverts et/ou recouverts partiellement.

14. Procédé selon au moins une des revendications précédentes, **caractérisé en ce que** le substrat (12) et/ou la couche extérieure (20) présentent une structure tridimensionnelle telle que la structure soit transférée sur les couches appliquées ensuite.

15. Procédé selon au moins une des revendications précédentes, **caractérisé en ce que** pour appliquer la seconde couche (17), on utilise des matériaux biorésorbables, en particulier du magnésium et/ou des matériaux visibles par radiographie, en particulier du tantale ou respectivement ses alliages.

16. Procédé selon au moins une des revendications précédentes, **caractérisé en ce que** la seconde couche (17) est la seule couche permettant de former un recouvrement monocouche (11) ou qu'au moins une autre, en particulier plusieurs couches, sont appliquées sur la seconde couche (17) pour former un recouvrement multicouche (11).
